(19) Europäisches Patentamt
European Patent Office
Office européen des brevets

(11) Veröffentlichungsnummer: **0 059 451**
**B1**

(12) # EUROPÄISCHE PATENTSCHRIFT

(45) Veröffentlichungstag der Patentschrift: **31.07.85**

(51) Int. Cl.⁴: **C 07 D 285/16**, C 08 F 4/40, C 08 F 4/32, A 61 K 6/08, A 61 K 6/10

(21) Anmeldenummer: **82101493.3**

(22) Anmeldetag: **26.02.82**

(54) **1,2,6-Thiadiazin-3,5-dion-1,1-dioxide und ihre Verwendung.**

(30) Priorität: **27.02.81 DE 3107577**

(43) Veröffentlichungstag der Anmeldung: **08.09.82 Patentblatt 82/36**

(45) Bekanntmachung des Hinweises auf die Patenterteilung: **31.07.85 Patentblatt 85/31**

(84) Benannte Vertragsstaaten: **AT BE CH DE FR GB IT LI LU NL SE**

(56) Entgegenhaltungen:
**DE - B - 1 090 224**
**US - A - 2 686 775**
**US - A - 3 207 815**

(73) Patentinhaber: **Espe Fabrik Pharmazeutischer Präparate GmbH, D-8031 Seefeld / Obb (DE)**

(72) Erfinder: **Schmitt, Werner, Dr., Prinzenweg 10, D-8130 Starnberg (DE)**
Erfinder: **Hübner, Heinz-Joachim, Dr., Moosbichlweg 16, D-8031 Wörthsee/Steinebach (DE)**
Erfinder: **Purrmann, Robert, Dr., Riemerschmidstrasse 18, D-8130 Starnberg (DE)**
Erfinder: **Burger, Bernd, Hirtenweg 4a, D-8031 Hechendorf (DE)**
Erfinder: **Jochum, Peter, Dr., Pointweg 5, D-8031 Hechendorf (DE)**

(74) Vertreter: **Abitz, Walter, Dr.-Ing. et al, Abitz, Morf, Gritschneder, Freiherr von Wittgenstein Postfach 86 01 09, D-8000 München 86 (DE)**

EP 0 059 451 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Aus USA-Patentschrift 2 956 997 sind 1,2,6-Thiadiazin-3,5-dion-1,1-dioxide (Malonylsulfamide) bekannt, bei denen der Substituent an dem einen Ring-Stickstoffatom ein aromatischer oder araliphatischer Kohlenwasserstoffrest sein muß. Die dort beschriebenen Verbindungen sollen pharmazeutische Eigenschaften, insbesondere eine antiphlogistische, antipyretische und analgetische Wirksamkeit aufweisen und sollen daher für die Behandlung von rheumatischen Beschwerden geeignet sein.

In der deutschen Auslegeschrift 1 495 520 ist ein Verfahren zum Polymerisieren von Acrylsäureestern, Methacrylsäureestern, Acrylnitril, Vinylacetat oder Styrol in Gegenwart von organischen Peroxiden erläutert, bei dem man als Beschleuniger unter anderem Barbitursäuren einsetzen kann. Zusätzlich kann man außerdem eine Verbindung einsetzen, die ionogen gebundenes Halogen enthält, und/oder eine Kupferverbindung. Das dort beschriebene Verfahren soll beispielsweise Anwendung finden bei der Herstellung von Abdruckmassen, Füllmassen, Prothesen, Einbettungen und Fixierungen auf zahnärztlichem und zahntechnischem Gebiet.

Gegenstand der Erfindung sind 1,2,6-Thiadiazin-3,5-dion-1,1-dioxide der allgemeinen Formel

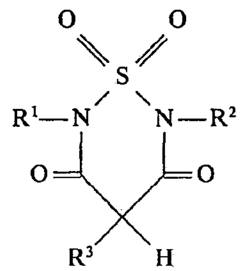

(I)

worin bedeuten

$R^1$ und $R^2$, die gleich oder verschieden sein können,
- $C_1$- bis $C_{18}$-Alkyl
- $C_1$- bis $C_{18}$-Alkyl, substituiert durch
  - $C_1$- bis $C_5$-Alkoxyl,
  - Halogen,
  - $C_1$- bis $C_5$-Alkoxycarbonyl oder
  - $C_4$- bis $C_7$-Cycloalkyl
- $C_3$- bis $C_5$-Alkenyl,
- $C_4$- bis $C_7$-Cycloalkyl, substituiert durch
  - $C_1$- bis $C_4$-Alkyl,
  - Halogen oder
  - $C_1$- bis $C_4$-Alkoxyl,

$R^3$
- $C_1$- bis $C_{18}$-Alkyl,
- $C_1$- bis $C_{18}$-Alkyl, substituiert durch
  - $C_1$- bis $C_5$-Alkoxyl,
  - Halogen
  - $C_1$- bis $C_5$-Alkoxycarbonyl,
  - $C_4$- bis $C_7$-Cycloalkyl,
  - Phenyl oder
  - Naphthyl,
- $C_3$- bis $C_5$-Alkenyl oder
- $C_4$- bis $C_7$-Cycloalkyl, substituiert durch
  - $C_1$- bis $C_4$-Alkyl,
  - Halogen oder
  - $C_1$- bis $C_4$-Alkoxyl,

sowie die Verwendung von 1,2,6-Thiadiazin-3,5-dion-1,1-dioxiden der allgemeinen Formel

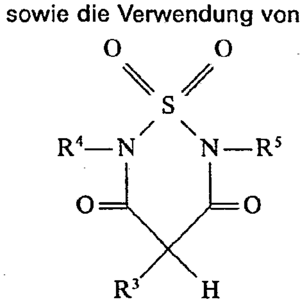

(II)

worin bedeuten

$R^4$ und $R^5$, die gleich oder verschieden sein können,
- $C_1$- bis $C_{18}$-Alkyl
- $C_1$- bis $C_{18}$-Alkyl, substituiert durch
  - $C_1$- bis $C_5$-Alkoxyl,
  - Halogen,
  - $C_1$- bis $C_5$-Alkoxycarbonyl,
  - $C_4$- bis $C_7$-Cycloalkyl,
  - Phenyl oder
  - Naphthyl,
- $C_3$- bis $C_5$-Alkenyl,
- $C_4$- bis $C_7$-Cycloalkyl,
- $C_4$- bis $C_7$-Cycloalkyl, substituiert durch
  - $C_1$- bis $C_4$-Alkyl,
  - Halogen oder
  - $C_1$- bis $C_4$-Alkoxyl,
- Phenyl,
- Phenyl, substituiert durch
  - $C_1$- bis $C_4$-Alkyl,
  - Halogen oder
  - $C_1$- bis $C_4$-Alkoxyl,
- Naphthyl oder
- Naphthyl, substituiert durch
  - $C_1$- bis $C_4$-Alkyl,
  - Halogen oder
  - $C_1$- bis $C_4$-Alkoxyl und

$R^3$
- $C_1$- bis $C_{18}$-Alkyl,
- $C_1$- bis $C_{18}$-Alkyl, substituiert durch
  - $C_1$- bis $C_5$-Alkoxyl,
  - Halogen,
  - $C_1$- bis $C_5$-Alkoxycarbonyl,
  - $C_4$- bis $C_7$-Cycloalkyl,
  - Phenyl oder
  - Naphthyl,
- $C_3$- bis $C_5$-Alkenyl oder
- $C_4$- bis $C_7$-Cycloalkyl, substituiert durch
  - $C_1$- bis $C_4$-Alkyl,
  - Halogen oder
  - $C_1$- bis $C_4$-Alkoxyl,

als Beschleuniger für die peroxidische Polymerisation von ethylenisch ungesättigten Verbindungen.

Die erfindungsgemäßen Verbindungen können nach einem der in der US-PS 2 956 997 beschriebenen Verfahren hergestellt werden, beispielsweise durch Umsetzung der entsprechenden Malonsäureverbindung oder einem Derivat einer solchen Verbindung mit einem entsprechend substituierten Sulfamid.

Die Verbindungen der allgemeinen Formeln I und II sind brauchbar als Beschleuniger für die durch Peroxo-Verbindungen bewirkte Polymerisation von ethylenisch ungesättigten Verbindungen, insbesondere von Acrylsäure-Verbindungen und Methacrylsäure-Verbindungen, vorzugsweise von deren Estern. Die Malonylsulfamide müssen so ausgewählt werden, daß sie im zu härtenden Monomeren hinreichend löslich sind.

Die Malonylsulfamide werden in Konzentrationen von 0,1 bis 15 Gew.-%, vorzugsweise von 0,1 bis 10 Gew.-%, bezogen auf die polymerisierbaren Monomeren, eingesetzt.

Zur weiteren Beschleunigung wird die Polymerisation vorzugsweise in Gegenwart von Schwermetallverbindungen und ionogenem Halogen oder Pseudohalogen durchgeführt. Als Peroxo-Katalysatoren werden zumeist organische Peroxide, wie Benzoylperoxid oder Lauroylperoxid, eingesetzt; als Schwermetall ist Kupfer, als Halogenid das Chlorion besonders geeignet. An Peroxo-Katalysatoren werden, gegebenenfalls zusammen mit einem Phlegmatisierungsmittel, 0,1 bis 10, vorzugsweise 0,5 bis 5 Gew.-%, an Schwermetall (geeigneterweise in Form löslicher, organischer Verbindungen) 1—200 ppm, vorzugsweise 10—100 ppm, und an (Pseudo)halogenidionen (geeigneterweise in Form eines löslichen Salzes) 0,01 bis 1 Gew.-%, bevorzugt 0,05 bis 0,5 Gew.-%, eingesetzt, jeweils bezogen auf den polymerisierbaren Anteil der Masse.

In üblicher Weise können die polymerisierbaren Massen noch Füllstoffe, Farbstoffe, Pigmente, Trübungsmittel und/oder Stabilisatoren gegen Licht- und Wärmeeinwirkung enthalten.

Für die Kaltpolymerisation von Monomeren, wie (Meth-)acrylsäureestern, haben die Verbindungen

der Formeln I und II den Vorteil gegenüber Barbitursäureverbindungen, daß die Temperatur während des Polymerisationsvorganges weniger hoch ansteigt.

Dies ist von besonderem Vorteil in der Dentalmedizin, wenn größere Substanzmengen im Munde polymerisiert werden müssen, wie dies z. B. bei der Herstellung von temporären Kronen und Brücken der Fall ist. Als polymerisierbare Monomere werden in der Dentaltechnik meist mono-, di- oder polyfunktionelle (Meth-)acrylsäureester verwendet, z. B. die Dimethacrylsäureester der deutschen Patentschrift 1 921 969 oder der deutschen Offenlegungsschrift 2 414 258.

Wie unten durch Vergleichsversuche gezeigt wird, werden unter Einsatz der erfindungsgemäßen Beschleuniger dentale, semipermanente Kronen und Brücken erhalten, die gegenüber den Materialien des Standes der Technik überlegene mechanische Eigenschaften aufweisen bei vergleichweise geringer Temperaturerhöhung während der Polymerisation.

Zur Verringerung des Polymerisationsschrumpfs und zur Erhöhung der mechanischen Festigkeit sowie zum Farbangleich an die natürlichen Zähne werden den Monomeren vor der Polymerisation Füllstoffe, Farbstoffe und/oder Fluoreszenzstoffe zugesetzt. Als Füllstoffe eignen sich unter anderem bereits fertig pigmentierte Polymethylmethacrylatperlen oder andere pulverisierte organische Polymerisate sowie auch anorganische Füllstoffe, insbesondere mikrofeine Füllstoffe, wie pyrogene Kieselsäure.

Die unter Einsatz der erfindungsgemäßen Verbindungen erhaltenen Polymerisate haben hohe Farbstabilität und gute mechanische Festigkeit.

Wenn einer der Reste $R^1$ bis $R^5$ unsubstituiertes $C_1$- bis $C_{18}$-Alkyl bedeutet, so kann dieser Rest gerade oder verzweigt sein und vorzugsweise 1—10 und insbesondere 1—6 Kohlenstoffatome aufweisen. Ganz besonders bevorzugt sind Alkylreste mit 3—6 Kohlenstoffatomen. Beispiele für niedrig-molekulare Alkylreste sind Methyl, Ethyl, Propyl, i-Propyl, n-Butyl, t-Butyl, s-Butyl, i-Butyl, n-Pentyl und Isoamyl.

Wenn einer der Reste $R^1$ bis $R^5$ einen substituierten $C_1$- bis $C_{18}$-Alkylrest darstellt, so weist der Alkylteil dieses Restes vorzugsweise die Anzahl von Kohlenstoffatomen auf, die oben für unsubstituiertes Alkyl angegeben sind.

Ist einer der Reste $R^1$ bis $R^5$ $C_1$- bis $C_5$-Alkoxy-$C_1$- bis $C_{18}$-alkyl oder $C_1$- bis $C_5$-Alkoxycarbonyl-$C_1$- bis $C_{18}$-alkyl, so enthält der Alkoxyrest vorzugsweise Methyl, Ethyl, Propyl, i-Propyl, n-Butyl, t-Butyl, i-Butyl, s-Butyl, n-Pentyl oder Isoamyl. Ist einer der Reste $R^1$ bis $R^5$ Halogen-$C_1$- bis $C_{18}$-alkyl, so wird unter Halogen Brom, Jod, Chlor oder Fluor verstanden.

Wenn einer der Reste $R^3$ bis $R^5$ Phenyl-$C_1$- bis $C_{18}$-alkyl bedeutet, so sind Benzyl und Phenylethyl bevorzugt.

Bedeutet einer der Reste $R^1$ bis $R^5$ $C_4$- bis $C_7$-Cycloalkyl-$C_1$- bis $C_{18}$-alkyl, so sind Cyclohexyl und Cyclopentyl als Cycloalkyl bevorzugt.

Bedeutet einer der Reste $R^1$ bis $R^5$ $C_3$- bis $C_5$-Alkenyl, so ist Allyl bevorzugt.

Wenn einer der Reste $R^4$ oder $R^5$ unsubstituiertes $C_4$- bis $C_7$-Cycloalkyl bedeutet, so sind Cyclopentyl und Cyclohexyl bevorzugt.

Bedeutet einer der Reste $R^1$ bis $R^5$ substituiertes $C_4$- bis $C_7$-Cycloalkyl, so sind die vorstehend angegebenen Cycloalkylreste bevorzugt, wobei der oder die Substituenten am Cycloalkylrest z. B. Methyl, Ethyl, Propyl, n-Butyl oder i-Butyl, Fluor, Brom, Jod, Chlor oder Methoxy sein können.

$R^4$ und $R^5$ können auch substituiertes Phenyl oder Naphthyl sein. Hier sind als Ringsubstituenten bevorzugt Methyl oder Methoxy.

Die Reste $R^1$ und $R^2$ bzw. die Reste $R^4$ und $R^5$ sind vorzugsweise identisch.

Als Einzelbeispiele von erfindungsgemäßen Malonylsulfamiden seien genannt:

| | |
|---|---|
| 2,6-Dimethyl-4-isobutyl-malonylsulfamid | (Fp. 59°C) |
| 2,6-Dibutyl-4-isobutyl-malonylsulfamid | (Fp. −3°C; $n_D^{20}$ 1,4711) |
| 2,6-Diisobutyl-4-propyl-malonylsulfamid | ($n_D^{20}$ 1,4707) |
| 2,6-Dibutyl-4-propyl-malonylsulfamid | ($n_D^{20}$ 1,4728) |
| 2,6-Dimethyl-4-ethyl-malonylsulfamid | (Fp. 50°C) |
| 2,6-Dioctyl-4-isobutyl-malonylsulfamid | ($n_D^{20}$ 1,4630) |

Die Vorteile der erfindungsgemäßen Malonylsulfamide bei der Kalthärtung zeigen die nachstehend aufgeführten Versuche:

Man löst 80 mg Bis-(1-phenyl-pentan-1,3-dionato)-Kupfer(II) und 4,8 g $\beta$-Phenylethyl-dibutylammoniumchlorid in 400 g 2,2-Bis[p-($\gamma$-hydroxy-propoxy)-phenyl]-propan-dimethacrylat und verknetet diese Lösung mit 85 g silanisierter, mikrofeiner Kieselsäure. Zu 1,36 g dieser Paste gibt man 40 mg 50%iges Benzoylperoxid (in Phthalat) und die in der Tabelle aufgeführte Barbitursäure bzw. das Malonylsulfamid hinzu. Die Menge an Beschleuniger wird durch einen Vorversuch so gewählt, daß der Abbindebeginn (Gelierungsbeginn) nach ca. 2,5 min erfolgt. Mit der aktivierten Mischung wird eine 1-ml-Kunststofform gefüllt und mit einem Trichter als Windschutz überdeckt. Durch die Trichteröffnung wird ein feines Thermoelement eingeführt und der Temperaturverlauf während der Erhärtung an einem geeigneten Meßinstrument verfolgt. Die Maximaltemperatur ist in der nachfolgenden Tabelle aufgeführt.

| Beschleuniger | Abbindebeginn (min/sec) | Abbindeende (min/sec) | Temperaturspitze (°C) |
|---|---|---|---|
| 1,5-Dimethyl-3-isobutyl-barbitursäure | 2'30" | 8' | 53 |
| 2,6-Dimethyl-4-isobutyl-malonylsulfamid | 2'30" | 5' | 45 |
| 2,6-Dibutyl-4-isobutyl-malonylsulfamid | 2'30" | 6' | 45 |

Ausgehend von Raumtemperatur ist im Falle der Barbitursäure die Temperaturerhöhung um mehr als ein Drittel größer als bei den erfindungsgemäßen Malonylsulfamiden. Diese geringere Temperaturerhöhung ist von besonderer Bedeutung in der Dentalmedizin, da thermische Belastungen bei der Polymerisation im Munde die durch das Beschleifen bereits stark beanspruchten Zahnstümpfe schädigen können.

Herstellungsbeispiel

208 g N,N'-Di-n-butylsulfamid werden in 400 ml Toluol gelöst. Bei Raumtemperatur werden unter Rühren 144 g frisch destilliertes Malonyldichlorid zugetropft. Es tritt Gasentwicklung bei leichter Wärmetönung ein. Nach dem Ende des Zutropfens wird für 7 h auf 70°C erwärmt. Das Lösungsmittel wird im Vakuum entfernt, der Rückstand in 2-n-Natronlauge gelöst und mit Toluol extrahiert.

Die wäßrige Phase wird mit Salzsäure angesäuert, mit Dichlormethan extrahiert, über Natriumsulfat getrocknet und lösungsmittelfrei gemacht. Das Rohprodukt wird im Vakuum destilliert; bei 0,4 mbar gehen zwischen 110°C und 115°C 175 g 2,6-Di-n-butyl-malonyl-sulfamid (I) über. Das Säureäquivalent bei der Titration mit Natriummethylat beträgt 275 (Theorie: 276).

30,5 g des Destillationsprodukts (I) werden in 100 ml Eisessig gelöst, mit 15,8 g Isobutyraldehyd versetzt und 5 h bei 80°C gehalten. Dann wird das Reaktionsgemisch in 1 l Wasser gegossen, das Gemisch mit Dichlormethan extrahiert, über Natriumsulfat getrocknet und das Lösungsmittel entfernt. Es wird erneut in 1 l Wasser gelöst und wie vorher extrahiert, getrocknet und zur Trockene gebracht. Man erhält 29 g 2,6-Di-n-butyl-4-isobutyliden-malonylsulfamid (II); das Säureäquivalent ist 333 (Theorie: 330); $n_D^{20} = 1,4887$.

10 g der Alkylidenverbindung (III) werden in 20 ml Tetrahydrofuran gelöst und zusammen mit ca. 2 ml Raney-Nickel in Stickstoffatmosphäre in einen Metallautoklaven gegeben. Nach Aufpressen von 50 bar Wasserstoff wird bei 100°C 1 min geschüttelt; der Druckabfall beträgt ca. 10 bar. Nach dem Abkühlen wird dekantiert, das Lösungsmittel entfernt und der Rückstand in 2-n Natronlauge aufgenommen. Nach Ausschütteln mit Toluol wird mit 2-n Salzsäure angesäuert, mit Dichlormethan extrahiert, über Natriumsulfat getrocknet und vom Lösungsmittel befreit. Das Rohprodukt wird durch Vakuumdestillation gereinigt (0,3 mbar, 130°C). Man erhält 3,5 g 2,6-Di-n-butyl-4-isobutyl-malonylsulfamid; Säureäquivalent 331 (Theorie: 330) $n_D^{20} = 1,4711$; Fp. −3°C.

$^1$H-NMR (60 MHz, rel. int. TMS):

0,8 − 1,13 $\delta$, Multiplett (m), 12 H
1,13 − 2,07 $\delta$, m, 11 H
3,63 − 4,15 $\delta$, m, 5 H.

Die weiteren Verbindungen werden in analoger Weise hergestellt:

| | |
|---|---|
| 2,6-Dimethyl-4-ethyl-malonylsulfamid | (Fp. 50°C) |
| 2,6-Dimethyl-4-isobutyl-malonylsulfamid | (Fp. 59°C) |
| 2,6-Diethyl-4-butyl-malonylsulfamid | ($n_D^{20} = 1,4761$) |
| 2,6-Dipropyl-4-ethyl-malonylsulfamid | ($n_D^{20} = 1,4760$) |
| 2,6-Dipropyl-4-propyl-malonylsulfamid | ($n_D^{20} = 1,4741$) |
| 2,6-Dibutyl-4-ethyl-malonylsulfamid | ($n_D^{20} = 1,4733$) |
| 2,6-Dibutyl-4-butyl-malonylsulfamid | ($n_D^{20} = 1,4729$) |

| | |
|---|---|
| 2,6-Diisobutyl-4-ethyl-malonylsulfamid | ($n_D^{20}$ = 1,4719) |
| 2,6-Diisobutyl-4-butyl-malonylsulfamid | (Fp. 31°C) |
| 2,6-Diisobutyl-4-benzyl-malonylsulfamid | (Fp. 73°C) |
| 2,6-Bis-(γ-methoxy-propyl)-4-isobutyl-malonylsulfamid | ($n_D^{20}$ = 1,4739) |
| 2,6-Dioctyl-4-isobutyl-malonylsulfamid | ($n_D^{20}$ = 1,4630) |
| 2,6-Dilauryl-4-isobutyl-malonylsulfamid | ($n_D^{20}$ = 1,4674) |
| 2,6-Dibutyl-4-lauryl-malonylsulfamid | ($n_D^{20}$ = 1,4622) |

## Anwendungsbeispiel 1 (Gießharz)

Es wird eine Lösung hergestellt aus:

| | |
|---|---|
| 400,0 g | 2,2-Bis[p-(γ-hydroxy-propoxy)-phenyl]-propan-dimethacrylat |
| 4,8 g | (β-Phenyl-ethyl)-dibutyl-ammonium-chlorid |
| 80,0 mg | Bis-(1-phenyl-pentan-1,3-dionato)-Kupfer (II) |

Zur Stabilisierung gegen frühzeitige Polymerisation und zur Standardisierung der Reaktivität werden dem Gemisch 50 ppm p-Methoxy-phenol zugesetzt; in einem geschlossenen Gefäß ist es mehrere Monate haltbar.

2 g dieser Lösung werden mit 80 mg 50%iger Benzoylperoxidpaste (in Phthalat) und 80 mg 2,6-Dimethyl-4-isobutyl-malonylsulfamid (1 : 1 mit Phthalat vermischt) rasch homogen vermischt. Die entstandene Masse ist ca. 2 min fließfähig und wird in eine zylindrische Metallform gegossen. Nach ca. 8 min ist unter geringer Wärmeentwicklung ein harter, glasklarer Formkörper entstanden.

## Anwendungsbeispiel 2 (Gießharz)

2 g der in Anwendungsbeispiel 1 hergestellten Lösung werden mit

| | |
|---|---|
| 80,0 mg | 20%iger Lauroylperoxidpaste (in Phthalat) und |
| 80,0 mg | 2,6-Dibutyl-4-isobutyl-malonyl-sulfamid (hergestellt nach dem Herstellungsbeispiel) |

homogen vermischt; die Masse bleibt etwa 2 min fließfähig. Das erhaltene Gemisch erhärtet unter geringer Wärmeentwicklung und ist zur Herstellung harter, glasklarer, farbstabiler Formkörper geeignet.

## Anwendungsbeispiel 3 (Dentalmasse)

Die in Anwendungsbeispiel 1 hergestellte Lösung wird mit 85 g silanisierter, mikrofeiner Kieselsäure (zahnähnlich eingefärbt) zu einer noch fließfähigen Paste verknetet.

| | |
|---|---|
| 680 mg | dieser Paste werden mit |
| 20 mg | 20%iger Lauroylperoxidpaste (in Phthalat) und |
| 20 mg | 2,6-Dibutyl-4-isobutyl-malonylsulfamid |

homogen vermischt. Die Mischung ist ca. 2,5 min verarbeitungsfähig und ist zur Herstellung von Zahnersatzteilen geeignet.

## Anwendungsbeispiel 4 (Herstellung einer semipermanenten Brücke)

1,36 g der aktivierten Mischung nach Anwendungsbeispiel 3 werden mit einer Spritze in einen Alginat- oder Silikonabdruck eingebracht, der von der Präparation der Brückenpfeilerzähne gewonnen wurde und in dem eine tiefe Rille zwischen den Abdrücken der Pfeilerzähne eingeschnitten ist. Der gefüllte Abdruck wird in den Mund des Patienten eingesetzt und nach Beginn der Erhärtung (etwa 3—4 min nach Mischbeginn) mit dem Formkörper wieder aus dem Munde entfernt; während der Erhärtung tritt nur eine geringe Temperaturerhöhung auf.

Ca. 6 min nach Mischbeginn kann die semipermanente Brücke in üblicher Weise finiert werden. Man erhält ein kosmetisch gutes und mechanisch stabiles Zahnersatzteil von hoher Farbstabilität, das gegebenenfalls reparaturfähig ist.

Vergleichsversuch

Einige physikalische Werte des nach Anwendungsbeispiel 3 erhaltenen, erhärteten Materials für semipermanente Kronen und Brücken (nachfolgend als »(A)« bezeichnet, werden mit den Daten handelsüblicher Materialien verglichen.

Material (B) ist auf Epimin-Basis (»SCUTAN«, Fa. ESPE);
Material (C) auf Acrylat-Basis (»TRIM«, Fa. Bosworth).

| Material | Druckfestigkeit | Biegefestigkeit | Oberflächenhärte |
|---|---|---|---|
| (A) (erfindungsgemäß) | 240 MPa | 100 MPa | 120 MPa |
| (B) Epimin-Basis (Stand der Technik) | 75 MPa | 70 MPa | 85 MPa |
| (C) Acrylat-Basis (Stand der Technik) | 70 MPa | 60 MPa | 40 MPa |

**Patentansprüche für die Vertragsstaaten: BE, CH, DE, FR, GB, IT, LI, LU, NL, SE**

1. 1,2,6-Thiadiazin-3,5-dion-1,1-dioxide der allgemeinen Formel

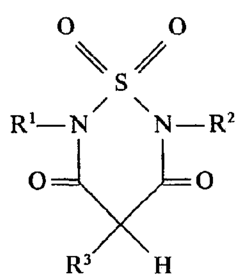

(I)

worin bedeuten

$R^1$ und $R^2$, die gleich oder verschieden sein können,
- $C_1$- bis $C_{18}$-Alkyl
- $C_1$- bis $C_{18}$-Alkyl, substituiert durch
  - $C_1$- bis $C_5$-Alkoxyl,
  - Halogen,
  - $C_1$- bis $C_5$-Alkoxycarbonyl oder
  - $C_4$- bis $C_7$-Cycloalkyl
- $C_3$- bis $C_5$-Alkenyl,
- $C_4$- bis $C_7$-Cycloalkyl, substituiert durch
  - $C_1$- bis $C_4$-Alkyl,
  - Halogen oder
  - $C_1$- bis $C_4$-Alkoxyl,

$R^3$
- $C_1$- bis $C_{18}$-Alkyl,
- $C_1$- bis $C_{18}$-Alkyl, substituiert durch
  - $C_1$- bis $C_5$-Alkoxyl,
  - Halogen
  - $C_1$- bis $C_5$-Alkoxycarbonyl,
  - $C_4$- bis $C_7$-Cycloalkyl,
  - Phenyl oder
  - Naphthyl,
- $C_3$- bis $C_5$-Alkenyl oder
- $C_4$- bis $C_7$-Cycloalkyl, substituiert durch
  - $C_1$- bis $C_4$-Alkyl,
  - Halogen oder
  - $C_1$- bis $C_4$-Alkoxyl.

2. Verbindungen nach Anspruch 1, dadurch gekennzeichnet, daß $R^1$, $R^2$ und/oder $R^3$ $C_1$- bis $C_8$-Alkyl oder $C_3$- bis $C_5$-Alkenyl bedeuten.

3. Verbindungen nach Anspruch 1, dadurch gekennzeichnet, daß $R^1$, $R^2$ und/oder $R^3$ $C_1$- bis $C_6$-Alkyl bedeuten.

4. Verbindungen nach einem der Ansprüche 1 bis 3, dadurch gekennzeichnet, daß $R^1$ und $R^2$ die gleiche Bedeutung haben.

5. Verbindungen nach Anspruch 1, dadurch gekennzeichnet, daß $R^1$, $R^2$ und $R^3$ $C_3$- bis $C_6$-Alkyl bedeuten.

6. Verwendung von 1,2,6-Thiadiazin-3,5-dion-1,1-dioxiden der allgemeinen Formel

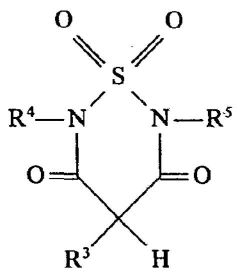

(II)

worin bedeuten

$R^4$ und $R^5$, die gleich oder verschieden sein können,
- $C_1$- bis $C_{18}$-Alkyl
- $C_1$- bis $C_{18}$-Alkyl, substituiert durch
  - $C_1$- bis $C_5$-Alkoxyl,
  - Halogen,
  - $C_1$- bis $C_5$-Alkoxycarbonyl,
  - $C_4$- bis $C_7$-Cycloalkyl,
  - Phenyl oder
  - Naphthyl,
- $C_3$- bis $C_5$-Alkenyl,
- $C_4$- bis $C_7$-Cycloalkyl,
- $C_4$- bis $C_7$-Cycloalkyl, substituiert durch
  - $C_1$- bis $C_4$-Alkyl,
  - Halogen oder
  - $C_1$- bis $C_4$-Alkoxyl,
- Phenyl,
- Phenyl, substituiert durch
  - $C_1$- bis $C_4$-Alkyl,
  - Halogen oder
  - $C_1$- bis $C_4$-Alkoxyl,
- Naphthyl oder
- Naphthyl, substituiert durch
  - $C_1$- bis $C_4$-Alkyl,
  - Halogen oder
  - $C_1$- bis $C_4$-Alkoxyl und

$R^3$
- $C_1$- bis $C_{18}$-Alkyl,
- $C_1$- bis $C_{18}$-Alkyl, substituiert durch
  - $C_1$- bis $C_5$-Alkoxyl,
  - Halogen,
  - $C_1$- bis $C_5$-Alkoxycarbonyl,
  - $C_4$- bis $C_7$-Cycloalkyl,
  - Phenyl oder
  - Naphthyl,
- $C_3$- bis $C_5$-Alkenyl oder
- $C_4$- bis $C_7$-Cycloalkyl, substituiert durch
  - $C_1$- bis $C_4$-Alkyl,
  - Halogen oder
  - $C_1$- bis $C_4$-Alkoxyl,

als Beschleuniger für die peroxidische Polymerisation von ethylenisch ungesättigten Verbindungen.

7. Verwendung nach Anspruch 6 als Beschleuniger für die peroxidische Polymerisation von Methacrylsäureverbindungen oder Acrylsäureverbindungen.

8. Verwendung nach Anspruch 6 oder 7 zusammen mit einer Schwermetallverbindung und einem Halogenid- oder Pseudohalogenidion.
9. Verwendung nach Anspruch 8 zusammen mit einer Kupferverbindung und dem Chloridion.
10. Verwendung nach einem der Ansprüche 6 bis 9 zur Herstellung von Dentalmassen.

**Patentansprüche für den Vertragsstaat: AT**

1. Verwendung von 1,2,6-Thiadiazin-3,5-dion-1,1-dioxiden der allgemeinen Formel

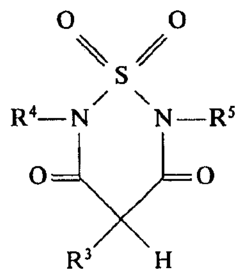

(II)

worin bedeuten

$R^4$ und $R^5$, die gleich oder verschieden sein können,
- $C_1$- bis $C_{18}$-Alkyl
- $C_1$- bis $C_{18}$-Alkyl, substituiert durch
  - $C_1$- bis $C_5$-Alkoxyl,
  - Halogen,
  - $C_1$- bis $C_5$-Alkoxycarbonyl,
  - $C_4$- bis $C_7$-Cycloalkyl,
  - Phenyl oder
  - Naphthyl,
- $C_3$- bis $C_5$-Alkenyl,
- $C_4$- bis $C_7$-Cycloalkyl,
- $C_4$- bis $C_7$-Cycloalkyl, substituiert durch
  - $C_1$- bis $C_4$-Alkyl,
  - Halogen oder
  - $C_1$- bis $C_4$-Alkoxyl,
- Phenyl,
- Phenyl, substituiert durch
  - $C_1$- bis $C_4$-Alkyl,
  - Halogen oder
  - $C_1$- bis $C_4$-Alkoxyl,
- Naphthyl oder
- Naphthyl, substituiert durch
  - $C_1$- bis $C_4$-Alkyl,
  - Halogen oder
  - $C_1$- bis $C_4$-Alkoxyl und

$R^3$
- $C_1$- bis $C_{18}$-Alkyl,
- $C_1$- bis $C_{18}$-Alkyl, substituiert durch
  - $C_1$- bis $C_5$-Alkoxyl,
  - Halogen,
  - $C_1$- bis $C_5$-Alkoxycarbonyl,
  - $C_4$- bis $C_7$-Cycloalkyl,
  - Phenyl oder
  - Naphthyl,
- $C_3$- bis $C_5$-Alkenyl oder
- $C_4$- bis $C_7$-Cycloalkyl, substituiert durch
  - $C_1$- bis $C_4$-Alkyl,
  - Halogen oder
  - $C_1$- bis $C_4$-Alkoxyl,

als Beschleuniger für die peroxidische Polymerisation von ethylenisch ungesättigten Verbindungen.

2. Verwendung nach Anspruch 1 als Beschleuniger für die peroxidische Polymerisation von Methacrylsäureverbindungen oder Acrylsäureverbindungen.

3. Verwendung nach Anspruch 1 oder 2 zusammen mit einer Schwermetallverbindung und einem Halogenid- oder Pseudohalogenidion.

4. Verwendung nach Anspruch 3 zusammen mit einer Kupferverbindung und dem Chloridion.

5. Verwendung nach einem der Ansprüche 1 bis 4 zur Herstellung von Dentalmassen.

**Claims for the Contracting States: BE, CH, DE, FR, GB, IT, LI, LU, NL, SE**

1. 1,2,6-Thiadiazine-3,5-dione-1,1-dioxides of the general formula

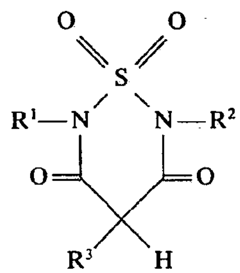

(I)

wherein

$R^1$ and $R^2$, which may be the same or different, stand for
- $C_1$ to $C_{18}$ alkyl,
- $C_1$ to $C_{18}$ alkyl substituted by
  - $C_1$ to $C_5$ alkoxyl,
  - halo,
  - $C_1$ to $C_5$ alkoxycarbonyl, or
  - $C_4$ to $C_7$ cycloalkyl,
- $C_3$ to $C_5$ alkenyl,
- $C_4$ to $C_7$ cycloalkyl substituted by
  - $C_1$ to $C_4$ alkyl,
  - halo, or
  - $C_1$ to $C_4$ alkoxyl;

$R^3$ stands for
- $C_1$ to $C_{18}$ alkyl,
- $C_1$ to $C_{18}$ alkyl substituted by
  - $C_1$ to $C_5$ alkoxyl,
  - halo,
  - $C_1$ to $C_5$ alkoxycarbonyl,
  - $C_4$ to $C_7$ cycloalkyl,
  - phenyl, or
  - naphthyl,
- $C_3$ to $C_5$ alkenyl, or
- $C_4$ to $C_7$ cycloalkyl substituted by
  - $C_1$ to $C_4$ alkyl,
  - halo, or
  - $C_1$ to $C_4$ alkoxyl.

2. Compounds according to claim 1, characterized in that $R^1$, $R^2$ and/or $R^3$ stand for $C_1$ to $C_8$ alkyl or $C_3$ to $C_5$ alkenyl.

3. Compounds according to claim 2, characterized in that $R^1$, $R^2$ and/or $R^3$ stand for $C_1$ to $C_6$ alkyl.

4. Compounds according to one of claims 1 to 3, characterized in that $R^1$ and $R^2$ have the same meaning.

5. Compounds according to claim 1, characterized in that $R^1$, $R^2$ and $R^3$ stand for $C_3$ to $C_6$ alkyl.

6. The use 1,2,6-thiadiazine-3,5-dione-1,1-dioxides of the general formula

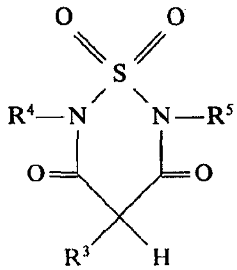

(II)

wherein

$R^4$ and $R^5$, which may be the same or different, stand for
- $C_1$ to $C_{18}$ alkyl,
- $C_1$ to $C_{18}$ alkyl substituted by
  - $C_1$ to $C_6$ alkoxyl,
  - halo,
  - $C_1$ to $C_5$ alkoxycarbonyl,
  - $C_4$ to $C_7$ cycloalkyl,
  - phenyl, or
  - naphthyl,
- $C_3$ to $C_5$ alkenyl,
- $C_4$ to $C_7$ cycloalkyl,
- $C_4$ to $C_7$ cycloalkyl substituted by
  - $C_1$ to $C_4$ alkyl,
  - halo, or
  - $C_1$ to $C_4$ alkoxyl,
- phenyl,
- phenyl substituted by
  - $C_1$ to $C_4$ alkyl,
  - halo, or
  - $C_1$ to $C_4$ alkoxyl,
- naphthyl, or
- naphthyl substituted by
  - $C_1$ to $C_4$ alkyl,
  - halo, or
  - $C_1$ to $C_4$ alkoxyl; and

$R^3$ stands for
- $C_1$ to $C_{18}$ alkyl,
- $C_1$ to $C_{18}$ alkyl substituted by
  - $C_1$ to $C_5$ alkoxyl,
  - halo,
  - $C_1$ to $C_5$ alkoxycarbonyl,
  - $C_4$ to $C_7$ cycloalkyl,
  - phenyl, or
  - naphthyl,
- $C_3$ to $C_5$ alkenyl, or
- $C_4$ to $C_7$ cycloalkyl substituted by
  - $C_1$ to $C_4$ alkyl,
  - halo, or
  - $C_1$ to $C_4$ alkoxyl,

as accelerators for the peroxidic polymerization of ethylenically unsaturated compounds.

7. The use according to claim 6 as accelerators for the peroxidic polymerization of methacrylic acid compounds or acrylic acid compounds.

8. The use according to claim 6 or 7 together with a heavy metal compound and a halide or pseudohalide ion.

9. The use according to claim 8 together with a copper compound and the chloride ion.

10. The use according to one of claims 6 to 9 for the preparation of dental compositions.

**Claims for the Contracting States: AT**

1. The use of 1,2,6-thiadiazine-3,5-dione-1,1-dioxides of the general formula

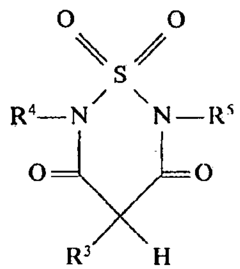

(II)

wherein

$R^4$ and $R^5$, which may be the same or different, stand for
$C_1$ to $C_{18}$ alkyl,
$C_1$ to $C_{18}$ alkyl substituted by
$C_1$ to $C_5$ alkoxyl,
halo,
$C_1$ to $C_5$ alkoxycarbonyl,
$C_4$ to $C_7$ cycloalkyl,
phenyl, or
naphthyl,
$C_3$ to $C_5$ alkenyl,
$C_4$ to $C_7$ cycloalkyl,
$C_4$ to $C_7$ cycloalkyl substituted by
$C_1$ to $C_4$ alkyl,
halo, or
$C_1$ to $C_4$ alkoxyl,
phenyl,
phenyl substituted by
$C_1$ to $C_4$ alkyl,
halo, or
$C_1$ to $C_4$ alkoxyl,
naphthyl, or
naphthyl substituted by
$C_1$ to $C_4$ alkyl,
halo, or
$C_1$ to $C_4$ alkoxyl; and
$R^3$ stands for
$C_1$ to $C_{18}$ alkyl,
$C_1$ to $C_{18}$ alkyl substituted by
$C_1$ to $C_5$ alkoxyl,
halo,
$C_1$ to $C_5$ alkoxycarbonyl,
$C_4$ to $C_7$ cycloalkyl,
phenyl, or
naphthyl,
$C_3$ to $C_5$ alkenyl, or
$C_4$ to $C_7$ cycloalkyl substituted by
$C_1$ to $C_4$ alkyl,
halo, or
$C_1$ to $C_4$ alkoxyl,

as accelerators for the peroxidic polymerization of ethylenically unsaturated compounds.

2. The use according to claim 1 as accelerators for the peroxidic polymerization of methacrylic acid compounds or acrylic acid compounds.

3. The use according to claim 1 or 2 together with a heavy metal compound and a halide or pseudo-halide ion.

4. The use according to claim 3 together with a copper compound and the chloride ion.

5. The use according to one of claims 1 to 4 for the preparation of dental compositions.

**Revendications pour les Etats contractants: BE, CH, DE, FR, GB, IT, LI, LU, NL, SE**

1. 1,1-dioxydes de 1,2,6-thiadiazine-3,5-diones de formule générale

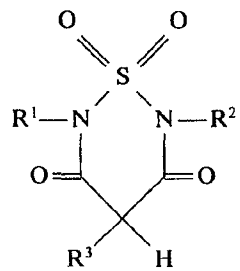

(I)

dans laquelle

$R^1$ et $R^2$, qui peuvent être identiques ou différents, représentent
des groupes alkyle en C 1–C 18,
des groupes alkyle en C 1–C 18, substitués par
des groupes alcoxy en C 1–C 5,
des halogènes,
des groupes (alcoxy en C 1–C 5)-carbonyle ou
cycloalkyle en C 4–C 7,
des groupes alcényle en C 3–C 5,
des groupes cycloalkyle en C 4–C 7, substitués par
des groupes alkyle en C 1–C 4,
des halogènes ou
des groupes alcoxy en C 1–C 4,
$R^3$ représente un groupe alkyle en C 1–C 18,
un groupe alkyle en C 1–C 18, substitué par
des groupes alcoxy en C 1–C 5,
des halogènes,
des groupes (alcoxy en C 1–C 5)-carbonyle,
des groupes cycloalkyle en C 4–C 7,
des groupes phényle ou naphtyle,
un groupe alcényle en C 3–C 5 ou bien
un groupe cycloalkyle en C 4–C 7, substitué par
des groupes alkyle en C 1–C 4,
des halogènes ou
des groupes alcoxy en C 1–C 4.

2. Composés selon la revendication 1, caractérisés en ce que $R^1$, $R^2$ et/ou $R^3$ représentent des groupes alkyle en C 1–C 8 ou alcényle en C 3–C 5.
3. Composés selon la revendication 2, caractérisés en ce que $R^1$, $R^2$ et/ou $R^3$ représentent des groupes alkyle en C 1–C 6.
4. Composés selon l'une des revendications 1 à 3, caractérisés en ce que $R^1$ et $R^2$ sont identiques.
5. Composés selon la revendication 1, caractérisés en ce que $R^1$, $R^2$ et $R^3$ représentent des groupes alkyle en C 3–C 6.
6. Utilisation des 1,1-dioxydes de 1,2,6-thiadiazine-3,5-diones de formule générale

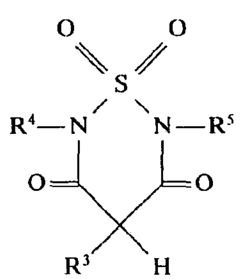

(II)

dans laquelle

$R^4$ et $R^5$, qui peuvent être identiques ou différents, représentent
des groupes alkyle en C 1–C 18,
des groupes alkyle en C 1–C 18, substitués par
des groupes alcoxy en C 1–C 5,
des halogènes,
des groupes (alcoxy en C 1–C 5)-carbonyle,
des groupes cycloalkyle en C 4–C 7,
des groupes phényle ou naphtyle,
des groupes alcényle en C 3–C 5,
des groupes cycloalkyle en C 4–C 7,
des groupes cycloalkyle en C 4–C 7, substitués par
des groupes alkyle en C 1–C 4,
des halogènes ou
des groupes alcoxy en C 1–C 4,
des groupes phényle,
des groupes phényle, substitués par
des groupes alkyle en C 1–C 4,
des halogènes ou
des groupes alcoxy en C 1–C 4,
des groupes naphtyle ou
des groupes naphtyle, substitués par
des groupes alkyle en C 1–C 4,
des halogènes ou
des groupes alcoxy en C 1–C 4, et
$R^3$ représente un groupe alkyle en C 1–C 18,
un groupe alkyle en C 1–C 18, substitué par
des groupes alcoxy en C 1–C 5,
des halogènes,
des groupes (alcoxy en C 1–C 5)-carbonyle,
des groupes cycloalkyle en C 4–C 7,
des groupes phényle ou naphtyle,
un groupe alcényle en C 3–C 5 ou
un groupe cycloalkyle en C 4–C 7, substitué par
des groupes alkyle en C 1–C 4,
des halogènes ou
des groupes alcoxy en C 1–C 4,

en tant qu'accélérateurs pour la polymérisation peroxydique de composés à insaturation éthylénique.

7. Utilisation selon la revendication 6, en tant qu'accélérateurs pour la polymérisation peroxydique de dérivés de l'acide méthacrylique ou de dérivés de l'acide acrylique.

8. Utilisation selon la revendication 6 ou 7, avec un composé de métal lourd ou un ion halogénure ou pseudo-halogénure.

9. Utilisation selon la revendication 8, avec un composé du cuivre et l'ion chlorure.

10. Utilisation selon l'une des revendications 6 à 9, pour la préparation de masses dentaires.

**Revendications pour l'Etat contractant: AT**

1. Utilisation des 1,1-dioxydes de 1,2,6-thiadiazine-3,5-diones de formule générale

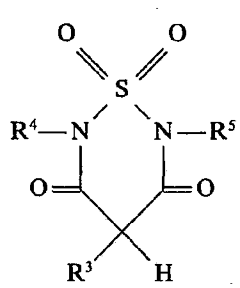

(II)

dans laquelle

$R^4$ et $R^5$, qui peuvent être identiques ou différents, représentent
des groupes alkyle en C 1—C 18,
des groupes alkyle en C 1—C 18, substitués par
des groupes alcoxy en C 1—C 5,
des halogènes,
des groupes (alcoxy en C 1—C 5)-carbonyle,
des groupes cycloalkyle en C 4—C 7,
des groupes phényle ou naphtyle,
des groupes alcényle en C 3—C 5,
des groupes cycloalkyle en C 4—C 7,
des groupes cycloalkyle en C 4—C 7, substitués par
des groupes alkyle en C 1—C 4,
des halogènes ou
des groupes alcoxy en C 1—C 4,
des groupes phényle,
des groupes phényle, substitués par
des groupes alcyle en C 1—C 4,
des halogènes ou
des groupes alcoxy en C 1—C 4,
des groupes naphtyle ou
naphtyle, substitués par
des groupes alkyle en C 1—C 4,
des halogènes ou
des groupes alcoxy en C 1—C 4, et
$R^3$ représente un groupe alkyle en C 1—C 18,
un groupe alkyle en C 1—C 18, substitué par
des groupes alcoxy en C 1—C 5,
des halogènes,
des groupes (alcoxy en C 1—C 5)-carbonyle,
des groupes cycloalkyle en C 4—C 7,
des groupes phényle ou naphtyle,
un groupe alcényle en C 3—C 5 ou
un groupe cycloalkyle en C 4—C 7, substitué par
des groupes alkyle en C 1—C 4,
des halogènes ou
des groupes alcoxy en C 1—C 4,

en tant qu'accélérateurs pour la polymérisation peroxydique de composés à insaturation éthylénique.

2. Utilisation selon la revendication 1, en tant qu'accélérateurs pour la polymérisation peroxydique de dérivés de l'acide méthacrylique ou de dérivés de l'acide acrylique.

3. Utilisation selon la revendication 1 ou 2, avec un composé de métal lourd et un ion halogénure ou pseudo-halogénure.

4. Utilisation selon la revendication 3, avec un composé du cuivre et l'ion chlorure.

5. Utilisation selon l'une des revendications 1 à 4, pour la préparation de masses dentaires.